# EUROPEAN PATENT APPLICATION

(11) **EP 1 857 458 A1**
(43) Date of publication of application: **21.11.2007**
(21) Application number: 06009316.8
(22) Date of filing: 05.05.2006
(51) Int. Cl.: C07D 473/16

(54) **Process for the preparation of abacavir**

(71) Applicant: SOLMAG S.p.A., 26837 Mulazzano LO (IT)
(72) Inventor: Rossi, Alessia, 20024 Garbagnate (Milano) (IT); Vecchio, Emilio, 20063 Cernusco Sul Naviglio (Milano) (IT); Pizzocaro, Roberta, 20024 Garbagnate (Milano) (IT); Bedeschi, Angelo, 20024 Garbagnate (Milano) (IT)
(74) Representative: Minoja, Fabrizio

(57) **Abstract**

A process for the preparation of Abacavir of formula **1** and its sulfate salt 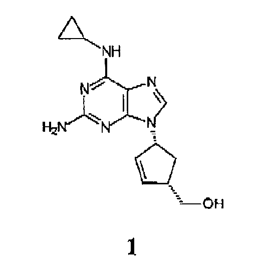 comprising the reaction of a compound of formula **6** with cyclopropyl amine.

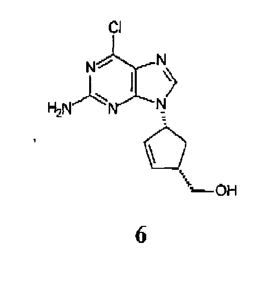

## Description

### Field of the invention

The present invention relates to an improved process for preparing Abacavir, a known antiviral agent.

### Background of the invention

Abacavir ((1S,4R)-4- [2- amino- 6- (cyclopropyl amino)- 9 H- purin- 9-yl]-2- cyclopentene- 1-methanol) of formula **1**, 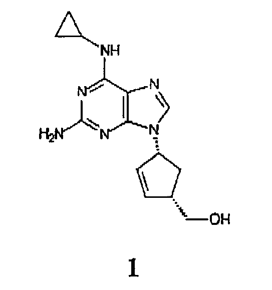 is a known antiviral agent and anti HIV reverse transcriptase inhibitor (Hervey P.S-, Perry C.M. in Drugs. 2000 Aug; 60(2):447-79 and cited references). Two main methods for the preparation of abacavir are disclosed in EP 434 450 and US 6,448,403. Both syntheses involve the use of a suitable di-halo aminopyrimidine starting material of formula **2** (scheme 1), 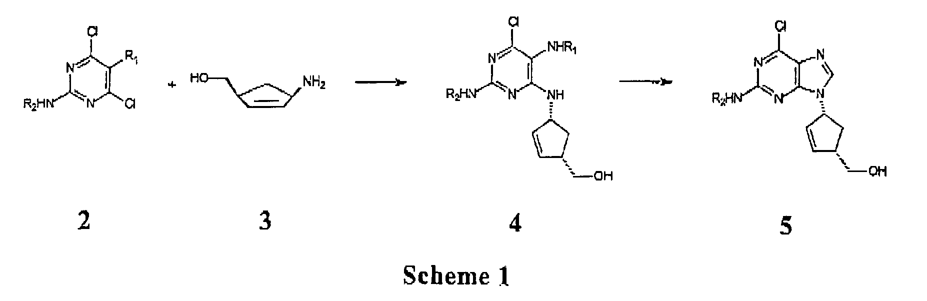 wherein R₁ is H, a nitro group, or a formylamino group and R₂ is a H or an acetyl group, that it is reacted with a suitable aminoalcohol of formula **3**, to give a compound of formula **4**, wherein R₂ is as described above, and R₁ is H or a formyl group, and then cyclized to yield a key intermediate compound of formula 5, wherein R₂ is H. The synthesis then continues with the displacement of the chlorine atom with cyclopropylamine to yield abacavir 1 as a free base.

The known syntheses are quite long, requiring a protection/deprotection sequence. In addition the introduction of the amino group requires the use of a nitration, or alternatively a diazotation reaction, generally considered dangerous.

An alternative process is reported in US 6,448,403, starting from a diamino-dihydroxy pyrimidine derivative which is subjected to a Vielsmaier reaction with POCl₃. However, said process requires anyway the use of dangerous reagents, such as POCl₃, and the addition of POCl₃ results in a strongly exothermic reaction. The precipitated complex/salts make stirring very difficult and the product is difficult to isolate.

Additional processes for the preparation of protected pyrimidine derivatives suitable for the synthesis of purine derivatives have been published, see for instance Harnden, M.R.; Wyatt et al.. J. Med. Chem, 1990, 33, 187, in which the dichloro diamino pyrimidine, prepared according to known methods, is formylated by means of a mixture of formic and acetic anhydride, to afford a fully protected compound of formula **2**, wherein R₁ is a formylamino group and R₂ is a formyl group. This method is more attractive starting from a readily and commercially available compound, using safer reaction conditions and affording pure products. The dichloro diformyl derivatives may be then reacted with a suitably protected chain. Nevertheless, in said document the method has been only exploited for the synthesis of a class of N-O derivatives, namely 9-(3-hydroxypropoxy)- and 9-[3-hydroxy-2-(hydroxymethyl)propoxy]purines and using fully protected linear, acyclic, side chains. Indeed, more recently, Ezzitouni, A et al., J. Chem. Soc., Perkin Trans. 1, 1997, 1073-1078, reported an application of the same synthesis to a carbo-bicyclic moiety, but still with fully protected OH moieties; therefore, after the cyclization, additional deprotection steps were necessary to remove the OH protecting groups, Furthermore, an additional step was necessary to remove also the formyl protecting group on the amino residue. All these deprotection steps impact on the synthesis length, on the yields, and consequently on the practicality of an industrial production.

Therefore there is still the need for a process for the preparation of compound (I) that is more practical and safer.

We have now surprisingly found that it is not only possible to perform a process able to obtain the key intermediate **5**, useful for abacavir synthesis, using an unprotected amino alcohol **3**, but also that the formyl protecting group of the amino group is spontaneously cleaved during the cyclization reaction to afford the fully unprotected 5, wherein R₂ is H.

### Summary of the invention

It is therefore an object of the present invention to provide an economical, safer and industrially practical process for the preparation of compound **6**, isolated as hydrochloride salt, key intermediate for the synthesis of abacavir of formula 1

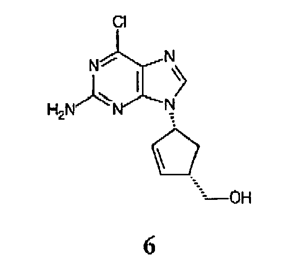

Said method avoids the prior art drawbacks and allows for a safe, easy, practical preparation of the compound of formula 6 and hence of abacavir.

Said process is new and comprises reacting a compound of formula 7, prepared according to Harnden, M. R. et al. J. Med. Chem, 1990, 33, 187, 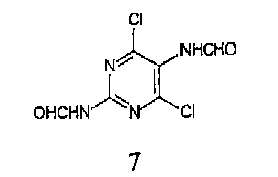 with a compound of formula 3, prepared according to known methods (see for instance Katagiri, N. et al. C. Tetrahedron Lett. 1989, 30, 1645-1648. Taylor, S.J.C. et al. Tetrahedron Asymmetry 1993, 4, 1117-1128). Further *in situ* cyclization of the above reaction mixture affords then compound of formula **6**. Chlorine substitution with cyclopropylamine then yields Abacavir.

### Detailed description of the invention

Accordingly, the present invention provides a method for production of Abacavir of formula 1 as free base and its sulfate salt 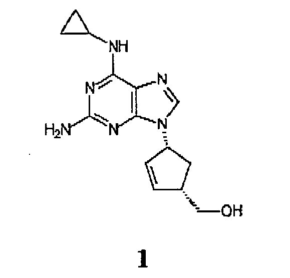 by reacting a compound of formula **6** with cyclopropyl amine

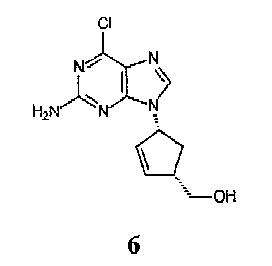

Said compound of formula **6** and its hydrochloride salt is prepared by a one-pot process comprising:
i) reaction of a compound of formula 7 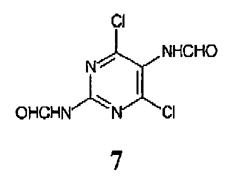 with a compound of formula 3 in a solvent in the presence of a base; 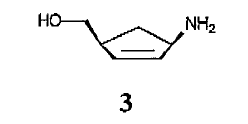
ii) reacting the crude from step i) with excess orthoformate or orthoacetate, in the presence of concentrated aqueous hydrohalogen acid, in an alcoholic solution.

Suitable solvents for step i) include C₁-C₄ linear or branched alcohols, optionally in mixtures with water, most preferably methanol, ethanol or isopropanol. Alcohols may be absolute or may contain variable amounts of water ranging from 1 to 20%. Examples of suitable bases include sodium or potassium bases such as, for instance NaOH, Na₂CO₃, NaHCO₃, NaOEt, NaOMe, or NaOt-bu; and KOH, K₂CO₃, KHCO₃, KOEt, KOMe, or KOt-bu. Preferred bases include NaOH, Na₂CO₃, NaHCO₃, KOH, K₂CO₃, or KHCO₃. Most preferred bases are Na₂CO₃, NaHCO₃, K₂CO₃ or KHCO₃, The reaction i) is carried out from room temperature to solvent reflux temperature. Typically, the reaction is run at or slightly below solvent reflux temperature. The reaction time may vary from few minutes to several days, most preferably from one hour to two days, and most preferably for a few hours.

Step ii) is carried out in presence of C₁-C₂ orthoformate, and in particular methyl or ethyl orthoformate. Aqueous hydrohalogen acids include HCl, HBr, and most preferably HCl, in a quantity ranging from 0.1 mol. equiv. to 10 mol equiv., and most preferably from 0.5 to 5 mol. equiv. Alcohols are defined as defined above for step i),

Temperatures may range from -10°C to solvent reflux temperature, preferably from 0°C to 60°C, and most preferably between 0°C and 40°C. The reaction time is preferably from a few hours to two days.

The desired compound 6 is finally isolated by filtration, washings with a solvent, as defined above in points i) and ii), and optionally drying. Alternatively, compound 6 may be used as such for the next step, without the drying step.

The following examples further illustrate the present invention.

### Example 1

### 4,6-Dichloro-2,5-diformamidopyrimidine (7).

Acetic anhydride (30 ml) was added to a solution of 4,6-dichloro-2,5-diaminopyrimidine (10 g), in formic acid (100 ml) at 0-5°C. After 15 min the solution was allowed to warm to room temperature and was stirred for a further 16 h. The solvent was then removed and the residue was coevaporated twice with toluene to afford the title compound (13 g), that was used without further purifications.

### Example 2

### (1S, 4R)-cis-4-[2-amino-6-chloro-9H-purin-9-yl]-2-cyclopentene-1-methanol (6), hydrochloride

(1S-cis)-4-amino-2cyclopentene-1-methanol, tartrate salt (55 g) was dissolved in ethanol (400 ml) in inert atmosphere, Sodium carbonate (48 g) was added. After 30 min 44 g of 4,6-dichloro-2,5-diformamidopyrimidinc (7) were added. The resulting mixture was then heated at gentle reflux. After 3 hours, the solvent was distilled off, and acetone (400 ml) was added. After 30 min reflux, the mixture is allowed to cool to 30°C, and the precipitate was filtered-off. The filtrate was evaporated to dryness, and the residue was taken-up with ethanol (200 ml). Triethylorthoformate (350 ml) and 35% HCl (35 ml) were added to the suspension, maintaining the temperature below 25°C. After 16 hours the suspension was filtered, and the solid was washed twice (50 ml) on the filter with ethanol, collected and dried, to give 30 g of the title product.

### Example 3

### (1S, 4R)-4-[2-amino-6-(cyclopropylamino)-9H-purin-9-yl]-2-cyclopentene-1-methanol (abacavir base, 1)

In a sealed reactor, isopropanol (200 ml) and cyclopropylamine (35 ml) were added to the compound obtained from example 2 (30 g). The reaction mixture was heated a 90-95°C for 12 hours. The mixture was allowed to cool at room temperature. A solution of sodium methylate 30% (18 g) (or an aqueous solution of sodium hydroxide, 27 g) was then added, and the solvent was evaporated, Isopropanol (200 ml) was added, and the precipitated salts were fltered-off. The filtrate was concentrated, and the oily residue was taken-up with acetone (150 ml). The resulting slurry was filtered, washed with acetone on the filter, and the collected solid was dried, yielding 25 g of the tile product as free base.

## Claims

1. A process for the preparation of Abacavir of formula 1 and its sulfate salt 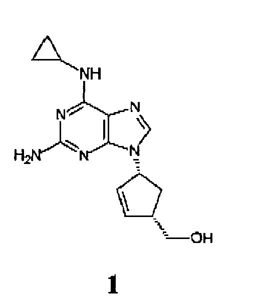 comprising the reaction of a compound of formula **6** with cyclopropyl amine.

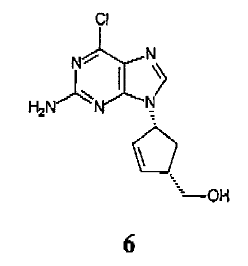

2. A process for the preparation of compound of formula **6**, as hydrochloride, comprising:
i) reaction of a compound of formula **7** 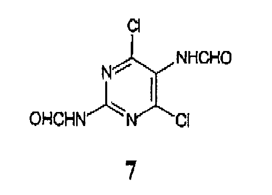 with a compound of formula **3** 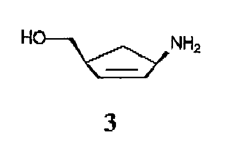 in a solvent in the presence of a base;
ii) reacting the crude from step i) with excess orthoformate or orthoacetate, in the presence of concentrated aqueous hydrohalogen aqueous acid, in an alcoholic solution.

3. A process according to claim 2 which is performed as a one-pot system.

4. A process according to claim 2 or 3 further comprising isolating the desired compound 6 by filtration, washings with a solvent, as defined above in points i) and ii), and optionally drying.
